# EUROPEAN PATENT SPECIFICATION

(11) **EP 2 117 491 B1**
(45) Date of publication and mention of the grant of the patent: **03.01.2018**
(21) Application number: 08710030.1
(22) Date of filing: 13.02.2008
(51) Int. Cl.: A61F 13/494, A61F 13/495, C09D 11/108, A61F 13/15

(54) **ABSORBENT ARTICLES COMPRISING AN INK COMPOSITION**
SAUGFÄHIGER ARTIKEL MIT EINER TINTENZUSAMMENSETZUNG
ARTICLE ABSORBANT COMPRENANT UNE COMPOSITION D'ENCRE

(30) Priority: 13.02.2007 EP 07102193
(43) Date of publication of application: 18.11.2009
(73) Proprietor: The Procter & Gamble Company, Cincinnati, OH 45202 (US)
(72) Inventor: LI, Wenbin, Mason, Ohio 45040 (US); WARNER, Alrick, Vincent, Loveland, Ohio 45140-6210 (US); PONOMARENKO, Ekaterina, Anatolyevna, 65812 Bad Soden (DE)
(74) Representative: L'Huillier, Florent Charles
(86) International application number: PCT/IB2008/050530
(87) International publication number: WO 2008/099360

(56) References cited:
- EP-A- 1 433 797
- EP-A- 1 642 943
- WO-A-03/010249
- WO-A-2006/007031
- US-A1- 2006 058 766

## Description

### FIELD OF THE INVENTION

The present invention relates to a process for making absorbent articles that comprise high barrier sheets that comprise an ink composition, applied in aqueous form, and processes for making such articles.

### BACKGROUND TO THE INVENTION

It has become practice in the sanitary article industry to provide articles like diapers and sanitary napkins with a printed backsheet or a diapers with a printed waistband portion, in particular to improve the appeal of the article. Separately, or in addition, such articles often comprise small printed marks needed during the production process, to register and phase the article's components. It recently has also become of interest to provide absorbent articles with printed information, which helps application of the articles.

Some of these articles comprise hydrophobic components, such as leg or barrier cuffs, or the recently developed anal/ genital cuffs, also referred to as topsheets with a large opening(s) such as described in for example EP1,201,212.

US2006/0058766A1 discloses an absorbent article comprising a topsheet with at least one opening to receive fecal matter, and comprising a genital coversheet which in use covers the genitals. The topsheet around the opening is elasticized. The articles may comprise a pair of longitudinally extending cuffs and the genital coversheet may be attached to these cuffs.

WO2006/0073031 discloses a process for making a nonwoven substrate having a vibrant graphic using a ink composition having a viscosity in the range of about 20 seconds to about 35 seconds using a Zahn 2 cup and comprising at least one solvent having an evaporation rate relative to n-butyl acetate of less than 0.8.

EP1,433,797A1 discloses a pigmented polymer composition comprising colorant particles, polymer particles comprised of polymerized units of phosphorus acid monomer and having a phosphorous group. The pigmented polymer composition is useful as paint or an ink. The application area mentioned for the coating of hard surface such as buildings or road.

WO03/010249A1 discloses an ink jet composition containing fluorochemical surfactants The ink has a low tendency to foam.

Hydrophobic backsheets of absorbent articles that require for example printed pictures typically are provided with a (plastic) film, which has a smooth surface that has a good affinity for the printing ink. Other hydrophobic materials of sanitary articles such as the cuffs may be colored with organic-solvent-based ink compositions, because the affinity of such sheet materials is better with such organic-solvent-based ink compositions.

Nevertheless, the inventors have found that it is difficult to print ink compositions on *high barrier nonwoven* materials, that have a fibrous surface with low affinity for ink; they found that it is difficult to provide a permanent print of good quality on nonwovens that have high barrier properties, whereof the ink is wearer friendly, non-irritable and does not impact the properties of the nonwoven sheet material. This may furthermore even be more difficult if these materials with ink compositions are in use in contact with bodily exudates such as blood and urine. Furthermore, when these nonwoven barrier materials are in use in contact with the skin of the user, it is important that no significant ink composition amounts are rubbed off onto the skin.

The inventors surprisingly found that specific water-based ink compositions comprising a polymeric fixation aid, e.g. a film-forming elastic polymer, for example applied as a dispersion of the polymer in water, and / or a specific surface tension reducing agent may be applied on the *high barrier nonwoven* sheets material to provide printed or coloured nonwoven sheets with excellent and permanent print and/ or colour quality, whilst (largely) maintaining the barrier properties. This is in particular surprisingly, because it is in general difficult to apply a water-based ink composition to highly hydrophobic barrier materials.

Furthermore, such water-based ink compositions can be formulated to be harmless to the skin of the user and safer to process.

### SUMMARY OF THE INVENTION

The present invention relates to a process for making an absorbent article comprising at least one cuff, wherein the cuff is a leg cuff, an anal cuff or a genital cuff, the cuff comprising a nonwoven sheet material that comprises an ink composition as indicated below. The method includes the steps of:
a) obtaining an hydrophobic nonwoven sheet material having a hydrostatic head (measured with 49 mN/m test liquid with the test method set out herein) of at least 15 mbar,;
b) obtaining an aqueous ink composition comprising water, a pigment, a fixation aid polymer which is an elastic film-forming polymer, and a surface tension reducing agent;
c) forming said nonwoven sheet material into a cuff and applying the cuff to an absorbent article, or applying said nonwoven sheet material to an absorbent article and forming it into a cuff, to obtain an absorbent article with a cuff;
d) applying said ink composition to said nonwoven sheet of step a) or to said cuff of step c), and
e) drying said nonwoven or cuff of step d).

The surface tension reducing agent preferably comprises a diol-based surfactant as described herein. Preferred nonwoven sheet materials and/ or cuffs comprise two or more nonwoven laminate layers, that are bonded together over a surface area of less than 40% of the total connecting area between said layers, each nonwoven laminate layer comprising two or more nonwoven webs that are laminated together and each nonwoven laminate layer comprising at least meltblown fibers.

### BRIEF DESCRIPTION OF THE FIGURES

Fig. 1 shows a perspective view of a preferred diaper of the invention, comprising a printed design, comprising the ink composition herein, in contracted or partially contracted state.
Fig. 2 shows a topview of the diaper of Figure 1, in contracted or partially contracted state.

### DETAILED DESCRIPTION OF THE INVENTION

As used herein, "absorbent article" means any article that can absorb body fluids and is suitable to be placed in close proximity to the genitals and/ or anus of the user (including in particular an adult or infant diaper and so-called training or pull-up pants, sanitary pads or napkins, panty-liners, adult incontinence pads)..

As used herein, the term "void space" is a cavity in or enclosed by the article present in at least the relaxed state, which serves to accept and contain bodily exudates such as fecal material, for example, at least 3 cm³ in relaxed state.

When used herein, "longitudinal" is the direction running substantially parallel to the maximum linear dimension of the component, typically to the longitudinal axis of the article, and includes directions within 30° of this parallel, when applicable.

The "lateral" or "transverse" direction is orthogonal to the longitudinal direction and in the same plan of the majority of the article and the longitudinal axis and includes directions within 30° of the orthogonal, when applicable.

As used herein, "elastic" or "elasticated" means typically, that the component or item, e.g. cuff, consists of or comprises an elastic material, which is elastic in at least one direction. "Non-elasticated" when used herein means that the component does not comprise any elastic material.

As used herein, "along" means "at least partially substantially parallel to and adjacent to". Adjacent includes "in close proximity with" and "in contact with".

As used herein, "opening in the cuff' or "opening in the topsheet" means an area in the topsheet or cuff that is large enough to receive fecal material, menses and/ or the genitals of the user, for example being at least 2 cm long or wide, or having a surface area of at least 2 cm², typically an area circumscribed by the topsheet, where the topsheet material is not present.

As used herein, a "nonwoven web" is a single web, whilst a "nonwoven layer" may comprise a multitude of nonwoven single webs, and a "nonwoven sheet" may comprise a multitude of nonwoven layers.

A nonwoven sheet or cuff herein is considered to have the parametric values described herein if it has such value at any part of the material. Preferred may be that more than one sample can be taken from the sheet or cuff, e.g. at least 3 or at least 5 samples, and that each sample has the parametric values as described herein.

### Absorbent articles

The absorbent article obtained by the process of the invention may be disposable absorbent articles, for typically single use. Preferred absorbent articles herein include sanitary napkins, panty-liners, adult incontinence pads or diapers, baby or infant diapers, including pull-on pants or training pants.

Preferred absorbent articles herein comprise, in addition to the cuff or cuffs described herein below, a backsheet that may be a liquid impervious, possibly vapour or air-pervious or breathable, backsheet, as known in the art. In preferred embodiments, the (liquid impervious) backsheet comprises a thin plastic film such as a thermoplastic film having for example a thickness of about 0.01 mm to about 0.05 mm. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964.

The backsheet, or any portion thereof, may be elastic, being elastically extendable in one or more directions.

The absorbent article typically comprises an absorbent core, to absorb bodily fluids received by said core, and said core may be positioned on said backsheet.

The absorbent article may comprise a core-cover sheet, e.g. positioned on said absorbent core and facing the skin of the user. In one embodiment herein, the cuff (described below) is in contact with the skin of the user and there underneath a topsheet or core-cover sheet may be placed, so that the cuff overlays the topsheet or core-cover sheet at least partially and the topsheet or core-cover sheet overlays the absorbent core. In one embodiment, a core cover sheet may be present overlaying the absorbent core and being (completely or partially) in contact with the skin of the user, whilst the cuffs do not overlay the absorbent core, but are positioned along or on the (longitudinal) edges of the core. The core cover sheet may have apertures, for example of an average largest diameter of from 0.1 mm to 5 mm. The topsheet may be a so-called apertured (formed) film, which may be a made of a liquid impervious material.

The backsheet may be attached or joined to said topsheet, the absorbent core, or any other element of the diaper by any attachment means known in the art. It may be possibly that the longitudinal side edges of the topsheet and backsheet are directly attached to one another, but that the longitudinal edges of the topsheet and the core are not attached to one another. The attachment means to attach the topsheet and the backsheet or any other parts or components of the article may include a uniform continuous layer of adhesive, a patterned layer of adhesive, or an array of separate lines, spirals, or spots of adhesive, such as disclosed in U.S.4,573,986. Adhesives that have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art. The absorbent core may comprise any absorbent material which is generally compressible, conformable, non-irritating to the wearer's skin, and capable of absorbing and retaining urine, such as comminuted wood pulp, creped cellulose wadding; melt blown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials; preferred may be absorbent cores which have an absorbent storage layer which comprises more than 80% by weight of the absorbent core content (e.g., excluding core wrap) of absorbent gelling material, and which is preferably free of absorbent cellulose material, pulp or airfelt. The absorbent article may also include a sub-layer disposed between the topsheet and the absorbent core, or between the cuff(s) and the topsheet or the absorbent core, capable of accepting and/or immobilizing bodily exudates, including fecal material. Suitable materials for use as a sub-layer may include large cell open foams, macro-porous compression resistant non woven highlofts, large size particulate forms of open and closed cell foams (macro and/or microporous), highloft non-wovens, polyolefin, polystyrene, polyurethane foams or particles, structures comprising a multiplicity of vertically oriented, preferably looped, strands of fibers, or preferably apertured formed films, as described above with respect to the genital coversheet. (As used herein, the term "microporous" refers to materials that are capable of transporting fluids by capillary action, but having a mean pore size of more than 50 microns. The term "macroporous" refers to materials having pores too large to effect capillary transport of fluid, generally having pores greater than about 0.5 mm (mean) in diameter and more specifically, having pores greater than about 1.0 mm (mean) in diameter, but typically less than 10 mm or even less than 6 mm (mean).

The absorbent article herein is preferably a disposable adult or infant diaper or training pants/ pull-up pants. Diapers or training pants of the invention may have side panels. The articles herein may comprise one or more pairs of elasticated leg cuffs that are not the cuffs of the present invention, but that (also) provide improved containment of liquids and other body exudates. Leg cuffs may also be referred to as leg bands, side flaps, barrier cuffs, or elastic cuffs, as described in; U.S. 3,860,003; U.S 4,808,178 and 4,909; U.S. 4,695,278 and 4,795,454. The diapers herein preferably have a fastening system, typically joined to the waistband, as known in the art. Preferred fastening systems comprise fastening tabs and landing zones, wherein the fastening tabs are attached or joined to the back region of the diaper and the landing zones are part of the front region of the diaper.

### Cuff(s)

The articles obtained by the process of the invention herein comprise at least one cuff comprising an ink composition as described herein. Said cuff may be a leg cuff, barrier cuff and/ or an anal and/ or genital cuff.

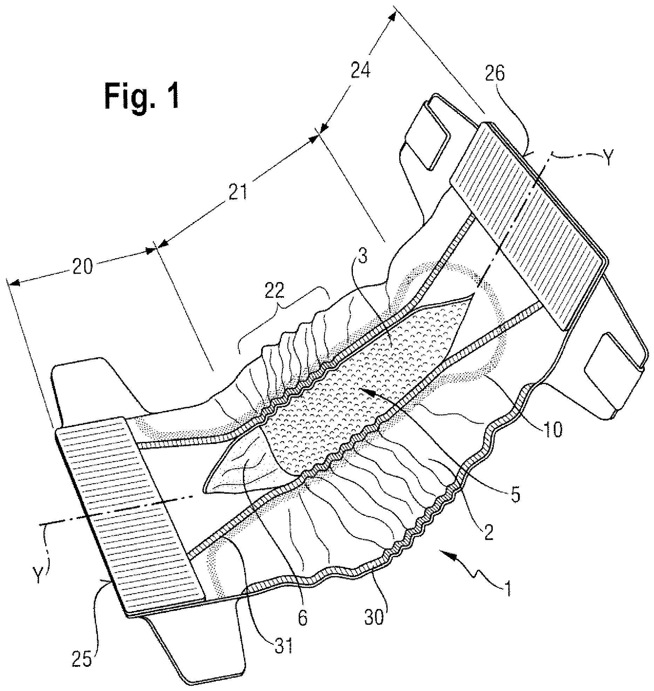

In one embodiment, the absorbent article of the invention comprises a barrier cuff and/ or leg cuff, and typically a pair of opposing barrier cuffs and/ or a pair of opposing leg cuffs, which comprise the ink composition herein. The ink composition may then be applied to the body facing surface of such a cuff and/ or the non-body-facing (opposite) surface of said cuff. Typically, the article comprises at least a pair of such opposing cuffs that are each positioned along one longitudinal side of the article and/ or the absorbent core, (and thus spaced apart from the y-axis of the article), as shown in Figure 1 as cuffs (30). The longest dimension or length of the barrier cuff or leg cuff herein and optionally of the elastic laminate portion (s), are typically parallel to the y-axis of the article and this is typically substantially parallel to the average direction of stretch of the elastic laminate portion and cuff. The width and length of the elastic laminate portions of the barrier and/ or leg cuffs herein will vary, typically depending on the exact dimensions of the cuff and/ or of the article. The two barrier cuff may be mirror images of one another mirrored in the y-axis. The pair of leg cuffs (25) may (also) be mirror images of one another mirrored in the y-axis.

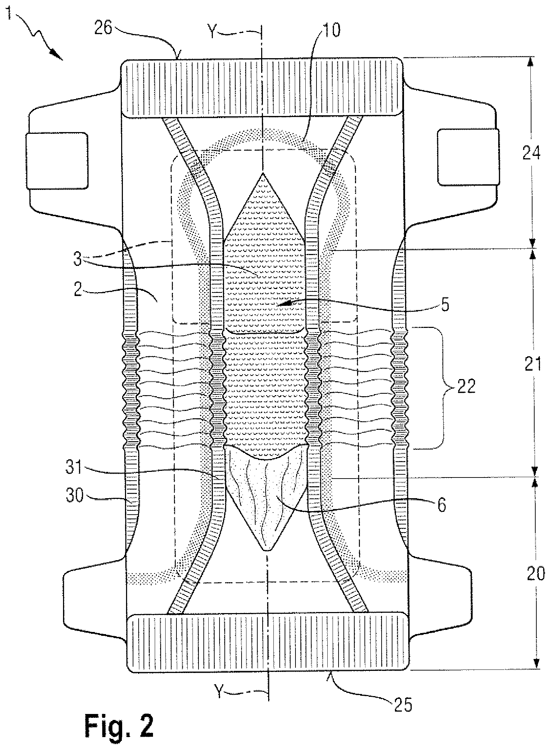

In one embodiment, the absorbent article comprises an anal/ and/ or genital cuff, comprising said ink composition, optionally in addition to the barrier and/ or leg cuff above, as for example shown in Figures 1 and 2. Such cuffs (2) are also referred to as topsheets with one or more openings. The anal and/ or genital cuff may be a sheet with one ore more opening that is or large enough to receive the exudates from the anus and/ or the genitals. It may be that this anal and genital cuff comprises one such opening that receives exudates from the anus and genitals. It may be preferred that the anal and/ or genital cuff has an average width that is the same or larger than the average width of the absorbent core and / or an average length that is the same or larger than the average length of the absorbent core. It may be that the anal and/ or genital cuff has an average width that is the same or larger than the average width of the backsheet.

The cuff(s) may comprise an elastic laminate portion, formed from an elastic material and said sheet material comprising the ink composition described herein, that itself is typically not elastically stretchable, said elastic laminate portion having preferably at least y-directional stretch (elongation). The elastic component or components may be comprised in a C-fold or Z-fold made of the sheet material of the cuff, and/ or it may be attached to the sheet material of the cuff and then covered on the opposite with a cover strip, or it may be non-covered on one surface. If the elastic component is not covered, it is preferably positioned on the surface of the cuff that in use is not in contact with the skin of the user.

The anal and/ or genital cuff herein may comprise one or more openings that are typically symmetrically positioned along part of the y-axis of the article and having at least two longitudinal opposing side edges, with there along one or more elastic components. Preferred opening (s) and elastic components are described herein below.

The cuff(s) may comprise other components, such as for example attachment means, such as adhesive.

The elastic materials used to elasticize the cuffs herein may for example be Lycra. It may for example be very thin, for example having a thickness or caliper (e.g. gauge) of up to about 200 microns, or even up to 150 microns or even up to 110 microns, or up to 100 microns and they may need to be at least 20 microns, or at least 40 microns, or at least 60 microns, or for example an average thickness of about 70 to 100 microns.

Preferred elastic materials used hereto may also include VFE-CD, available from Tredegar, and L-86, L-89, or L-90, available from Fulflex (Limerick, Ireland).

The cuff and/or the sheet material thereof, as useful in the invention herein, have high barrier properties, having a hydrostatic head before and/ or after application of the ink composition, as measured by the method set out herein, of at least 15 mbar. Preferably the hydrostatic head of the nonwoven sheet material or cuff after application of the ink composition is at least 70% of the hydrostatic head of said nonwoven sheet material or cuff prior to application of the ink composition, i.e. between 70% and 100% or preferably 80% to 100% as described herein after in more detail.

The nonwoven sheet material and typically the cuff has for example herein a hydrostatic head value (measured with a 49 mN/m test liquid with the method set out herein) of at least 15 mbar, or preferably at least 20 mbar, preferably at least 28 mbar, or even at least 30 mbar, or even at least 35 mbar, or even at least 40 mbar. A nonwoven sheet, or cuff herein is considered to have the above hydrostatic head values if it has this value at any part (whereof a sample is taken for the method set out herein) of the material, with the proviso that the measurement is done on a sample that does not comprise elastic material or edges attached to another material).

In one embodiment, the cuff and/or the nonwoven sheet material thereof as useful in the invention herein are hydrophobic, having prior to and/ or after application of the ink composition, a surface energy of between 26 and 40 mN/m, or between 28 and 32 mN/m or between 28 and 30 mN/m

In some embodiments herein, preferred nonwoven sheet materials for the cuffs herein comprise non-woven materials of natural fibers (e.g., wood or cotton fibers) and/ or synthetic fibers, and in some embodiments thermoplastic polymer fibers are preferred, such as selected from the group comprising: polyolefins, polyesters, polyurethanes, and polyamides, whereby it may be preferred that the thermoplastic polymer is a polyolefin, most preferably comprising polypropylene and/ or polyethylene and/ or polybutylene.

Most preferably, said nonwoven webs are formed from polyethylene, polypropylene and/ or polybutylene polymer fibers, or (a mixture of) fibers of a copolymers of polyethylene, polypropylene and/ or polybutylene; most preferred are polypropylene polymer fibers. Preferred may be polypropylene nonwoven webs and layers and sheets.

The fibers may be spun bond, carded, wet-laid, melt blown, and/ or hydro entangled, and/or otherwise processed as is known in the art. Preferred is that the cuff comprises a nonwoven sheet material that comprises one or more nonwoven laminate layers, each formed from laminated nonwoven webs, or possibly even that the nonwoven sheet material of the cuff is formed by attaching or partially attaching two or optionally more nonwoven laminate layers, each formed from laminated nonwoven webs. The laminated nonwoven layers may be laminates of a spunbond and a meltblown layer at least.

In one embodiment, the nonwoven sheet material of the cuff is formed from two (or more) nonwoven layers. Each nonwoven layer may comprise two or more nonwovens webs that are laminated together, whereby preferably said two or more nonwoven layers are only partially attached or bonded together, and thus not fully (100%) laminated together, for example with 60% or less, or 40% or less of the connecting surface area, or 20% or less of the connecting area (the connecting area being the area in the X-Y plane, in relaxed state, where both laminate nonwoven layers are present; e.g. only attached to one another along the edges of the connecting area of the two laminate nonwoven layers and optimally the area where elastic may be present. In one embodiment, the nonwoven layers are attached to one another along the side edges of each or one of the nonwoven webs, and optionally in the area where elastic component(s) are present, and the nonwoven layer comprises areas, e.g. of at least 0.5 cm², where both webs are present but not attached to one another. In one embodiment, the nonwoven sheet or the cuff comprises at least an area of 2.5 cm by 2.5 cm where the nonwoven layers are not attached to one another, and that does not comprise any edges or elastic component(s).

Preferred are nonwoven webs that comprise at least meltblown fibers. In one embodiment, each nonwoven layer comprises meltblown fibers, present at a weight level of at least 5 g/ m² by weight of the nonwoven layer, or for example at least 5.7 g/ m², or at least 7 g/ m². In one embodiment, or in the embodiment above where the layers comprise meltblown fibers, it may be preferred that the basis weight of the nonwoven sheet is 45 g/ m² or less and/ or it may be preferred that the basis weight of each of the nonwoven layers present in said nonwoven sheet is 24 g/m² or less, or 22g/m² or less.

It may also be beneficial that the sheet with the ink composition comprises nonwoven webs comprising so-called nanofibers, having fibers of an average fiber diameter of less than 1 mircon. For example, the sheet may comprise a nonwoven layer that comprises a spunbond nonwoven web and a nano fiber nonwoven web (for example with average fibre diameter of 0.6 micron), for example of 13.5gsm SB +3.1 gsm NF; or a nonwoven layer comprising a spunbond wen and a Nano fiber web of respectively 1.65gsm and 10gsm (whereby with 0.4micron nanofiber diameter average) with very similar properties.

Preferred are herein are: a nonwoven sheet comprising a 17 or 22 gsm (g/m²) SMMMS nonwoven layer attached to (but not laminated to) another 17 or 22 gsm SMMMS nonwoven layer (whereof for example the meltblown level of each layer is 5.7 or 7.3 gsm respectively), including a nonwoven sheet comprising 22 gsm SMMMS nonwoven layer, with for example 7.3gsm meltblown fibers, attached to 17 gsm SMMMS or SMMS nonwoven layer, comprising for example 5.7 gsm meltblown fibers; a nonwoven sheet comprising a 17 gsm or 22 gsm SMS or SNS nonwoven layer, attached to another 17 gsm or 22 gsm SNS or SMS nonwoven layer; a 17 or 22 gsm SMMS nonwoven layer attached to a 17 or 22 gsm SMMS or SMMMS nonwoven layer (comprising for example 3 gsm or 5.7 gsm (for SMMS) or 7.3 gsm meltblown fibers per layer).

It may also be preferred that the cuff comprises ingredients, which reduce friction between the wearer's skin and the cuff. Hereto, the cuff may (in addition to the ink composition) for example comprise a lotion, a fine powder, such as talcum powder, or wax.

The cuff may be treated with an agent to reduce its surface energy and/ or with a masking facilitating agent.

For example useful agent include fluorocarbons as described in U.S. Patent 5,876,753, issued to Timmons et al. on March 2, 1999; U.S. Patent 5,888,591 issued to Gleason et al. on March 30, 1999; U.S. Patent 6,045,877 issued to Gleason et al. on April 4, 2000. Other agents include silicone. Useful agents are described in WO2006/089183. Preferred agents may be selected from the group comprising fluorocarbons, siloxanes, polysiloxanes, preferably including fluorinated monomers and fluorinated polymers, including hexafluoroethylene, hexafluoropropylene and vinyl fluoride and vinylidene fluoride, fluoroacrylate and fluoromethacrylate.

The (anal and/ or vaginal) cuff is typically a barrier sheet that reduces the re-wetting through the sheet of the skin by any bodily exudates, e.g. menses or feces that may in use be present underneath the sheet. It may also form a visual barrier, so that bodily exudates stored under the cuff are less visible to the user or caregiver. It may thereto obtain also masking facilitating agents mentioned above, or other well-known masking compounds, or opacifying agents, including titanium dioxide.

In one embodiment, for example shown in Figures 1 and 2, where the cuff (2) herein is an anal and genital cuff, it may comprise at least one opening that is large enough to receive feces and allow it to pass to a void space underneath (5) said cuff. A single opening for the reception of at least fecal material may be preferred. The opening may be in the form of a slit opening. The opening is preferably present in (part of) the front region (20) of the cuff (in use towards the front of the user) and in (part of) the back region (24) of the cuff, each being 1/3 of the total length of the cuff in relaxed, contracted state. Preferably, the lit opening has a longitudinal dimension (length) substantially parallel to the longitudinal axis of the absorbent article. The opening may be configured such that from 20% to 40%, or more preferably from 20% to 30% of the length of the opening (in fully stretched state) extends from the transverse axis of the cuff towards the front edge of the cuff, and the remaining percentage extends towards the back edge of the cuff.

In one embodiment the opening is in the form of a slit opening with substantially parallel longitudinal side edges, which are connected in the front and back by V-shaped or rounded V-shaped front and back edges.

The average width of the opening herein, in 75 % stretched state, depends on the article, but may for example be from 5% to 30%, or more preferably 10% to 25%, of the average width of the cuff (including the opening, in fully stretched state).

The anal and/ or genital cuff (2) herein may comprise elastic components (elastics) (31) in the form of at least one pair of elastic bands or strands, one on either longitudinal side of said opening of the cuff, and preferably extending from said longitudinal side edges of the opening(s) towards or to the front edge and/or back edge of the cuff. The elastic bands or strands may thus be longer than the opening. Preferred is that the front end portions of two opposing primary elastics bend away from one another (in the plane of the cuff), so that the distance between the end edges of the opposing front end portions of two opposing elastics is larger that the distance between the centre portions of two opposing elastics, and equally, the distance between the end edges of the opposing back end portions of two opposing elastics is larger that the distance between the centre portions of two opposing elastics.

Then, each front end portion of an elastic band or strand has typically an angle with a longitudinal line through the centre of the elastic band or strand and parallel to the longitudinal axis of the cuff, said angle being between 10° and 45°, or preferably between 17° to 45°, or even more preferably between 20° and 35°.

Then, the back end portion of each of elastic band or strand has also preferably an angle with a longitudinal line through the centre of the elastic band or strand and parallel to the longitudinal axis of the cuff, said angle being between 10° and 50°, or preferably between 17° to 45°, or even more preferably between 25° and 45° (in fully stretched state).

The anal and/ or genital cuff may comprise a genital coversheet, for example as shown in Figures 1 and 2 as genital coversheet (6), that is present above, in or under the opening in the cuff (2) and above the absorbent core (3) The genital coversheet may (in fully stretched flat state) cover the front 10% to 50% of the maximum length of the opening, preferred 10% to 30%, or more preferably 13% to 28% or even more preferably 17% to 27%. In other words, in one embodiment, at the most 50% of the maximum length of the opening may be 'covered' by the longest part of the genital coversheet, but at least 10% of the maximum length of the opening is covered by the longest part of the genital coversheet. The genital coversheet may be hydrophilic and/ or urine-permeable, and/ or it may comprise apertures to pass urine.

In another embodiment the article may have two barrier cuffs or leg cuffs, as described herein, and a genital coversheet may then be attached to or placed over said pair of cuffs in the front region of the article, and thus covering part of the underlying core in the front region of the article. This sheet will then receive the genitals in use and protect the genitals from soiling, e.g. by fecal material.

The ink composition may be applied in any pattern to the cuff(s), such as for example the ink pattern (10) shown in Figures 1 and 2. It may be applied to all of the cuff area or to a part thereof, e.g. in a pattern, or to a portion of the cuff where the elastic material (e.g. bands, strands) are present, or to a portion of the cuff where the elastic material is not present. It may have an area, for example the area (22) shown in Figures 1 and 2, where the ink composition (10) is substantially parallel and/ or adjacent or along and/ or on top of the elastics (31) that may be present in the nowoven sheet material or cuff (2).

The ink composition may also be applied to materials or components of the absorbent article other than the nonwoven sheet material with barrier properties as described herein, e.g. to the front waistband (25) or back waistband (26).

### Ink composition

The ink composition herein is applied as an aqueous composition. The inventors found surprisingly that such ink aqueous compositions can be applied to high barrier nonwovens and/ or highly hydrophobic nonwovens, as described herein, despite the highly hydrophobic, barrier nature of these materials.

The aqueous ink composition comprises an aqueous fixation aid polymer component. The fixation aid polymer, as described herein (typically applied in the form of a dispersion in water) may be present in the aqueous ink composition at a level of for example 10% to 80% by weight of the aqueous ink composition, or from 15% to 50% or 20% to 40% by weight.

The surface tension reducing agent, as described herein, may be present at a level of for example 3% to 60% by weight of the aqueous ink composition, or from 5% to 50% or 10% to 40% by weight of the aqueous ink composition.

The aqueous ink composition to be applied may comprise for example at least 20% by weight, or at least 30%, or at least 45% or at least 50% by weight of water, prior to application. After application, the nonwoven sheet material, cuff or absorbent article is typically dried and most or all of the water is removed, typically such that the ink composition on the nonwoven sheet, after drying, comprises less than 5% by weight of the ink composition of water, or less than 3%, or less than 1% by weight.

After drying, the ink composition on the nonwoven sheet material or cuff may comprise for example from 5% to 50% of the polymer described herein, or for example from 10% to 40% by weight or from 15% to 35% by weight.

After drying, the ink composition on the nonwoven sheet material or cuff may comprise for example from 5% to 50% of the surface tension reducing agent or from 10% to 40% by weight or from 15% to 35% by weight.

It may be possible that the ink composition comprises less than 10% or less than 5% or less than 3% by weight of organic solvents, or no organic solvents (i.e. organic solvents that may be solvents for the pigment or the other components of the composition herein). Such solvents may include alcohols, heptane, n-propyl acetate, methyl ethyl ketone, methyl isobutyl ketone, toluene and/ or ethyl acetate, isopropyl acetate, N-butyl acetate and mixtures thereof. In one embodiment no organic solvents, for example none of the ones listed above, are present in the ink composition prior to and after application of the ink composition to the nonwoven sheet.

The composition may suitably comprise at least a pigment. The pigment may be present in any form, shape and quantity. Preferred may be that the pigment is present at a levels of from 0.5% to 40% by weight of the aqueous composition (prior to application), or for example from 1% to 30% by weight. Preferred may be that the pigment is in the form of small particles, having an average particle size of less than 1 micron, or for example from 0.01 - 0.20 micron.

The ink composition comprises a fixation aid polymer (including homopolymers, copolymers and blockpolymers etc.). The polymer herein is preferably applied in the form of aqueous dispersion of said polymer. This polymer is an elastic film-forming polymer, applied to the cuff or sheet material thereof as a solution in water or dispersion in water.

It is believed that such polymers may act as a binder of the pigment to the sheet material herein, providing homogeneous binding and protection of the pigment. It is believed that they may form a film that keeps the pigment on the sheet material.

The elastomeric film-forming polymer herein may be any polymer that is capable of forming a film on a nonwoven surface, at any temperature that occurs during application of the ink composition, e.g. between the process temperature or product-use temperature, for example at a temperature from 200°C, or 150°C, or 100°C to 20°C, or as low as 0°C (if cooling takes place during the process), by any film forming method known in the art, for example when being applied (e.g. as a spray) from a solution, dispersion or as hotmelt onto a flat nonwoven surface (with or without submitted it to a subsequent heat treatment step, e.g. curing or annealing step).

The polymer herein may be an elastomeric polymer, which typically means that the polymer, when deformed by stress, partially or completely recovers its mechanical properties when de deformation stress is removed. Preferred are hydrocarbon-based elastomeric, film-forming polymers.

Preferred herein are fixation aid polymers, e.g. being also elastic film forming polymers, which not only are believed to be binders for the pigment but that are also able to reduce the surface tension of the ink composition.

The ink composition herein comprises suitably a surface tension reducing agent e.g. that may be capable of reducing the surface tension of the aqueous ink composition, compared to the aqueous ink composition not comprising said agent, with at least 5mN/m, or at least 8mN/m, preferably at least mN/m, or even at least 12 mN/m. The resulting aqueous ink composition comprising the agent may have a surface tension of less than 36 mN/m, or less than 34 mN/m, or for example 30 mN/m or less, or between 28 and 30 mN/m.

Preferred surface reducing agents include dioctyl sodium sulfosuccinate or derivatives thereof, ethoxylated glycols, sorbitan esters and/ or acetylenic diol-based surfactants and mixtures thereof. The acetylenic diol-based surfactants may include alkoxylated (e.g..ethoxylated) acetylenic diols, including acetylenic glycols, and derivatives thereof, including alkyl branched derivatives thereof, e.g. with one or more methyl side-groups. The may for example have an alkoxylation (e.g. ethoxyaltion) degree of 2-50, preferably 4-10, or 4-8.

The compositions herein may for example comprise ethoxylated 2,5,8,11 tetramethyl-6 dodecyne 5,8 diols and derivatives thereof.

Suitable ink compositions comprising the diol-surfactant and/ or polymeric fixation aid component described herein are available from INX International Ink Co., Chicago, IL, USA.

Other additives may be optionally added to the aqueous ink composition. These additives may include, but are not limited to wetting agents, dispersants, anti-foamers, buffering agents, and surfactants other than described above. Generally, the amount of each of these additives would be about 0.0001 to 10%, more preferably 0.0001 to 5% by weight, based upon the total weight of the aqueous solution. Examples of surfactants include silicone surfactants, poly(alkylene oxide) surfactants, and fluorochemical surfactants. Suitable non-ionic surfactants for use in the aqueous solution include, but are not limited to glycols, ethoxylated glycols, sorbitan esters and mixtures thereof.

The ink composition may be formulated such that the nonwoven sheet material with the ink composition has substantially, e.g. between 70% and 100%, the same surface energy as the nonwoven sheet material not comprising said ink composition (e.g prior to applying said ink composition).

The optical density of the nonwoven sheet material or cuff with the ink composition as measured with the method set out herein may for example be at least 0.15 and more preferred at least 0.3 or at least 0.4.

The nonwoven sheet material or cuff comprising the ink composition herein may have a rub-off value of Delta E (CIE) of less than 4.0, preferably less than 3.5 or preferably less than 3.0, as measured by the method set out below.

The ink composition herein may comprise any colorant or pigment, including for example pigments with the colour index numbers (CI): Yellow 74, CI 11741; Red2- CI 12310; Blue 15:3, CI74160; Black 7 CI 77266,; Green 7, CI 74260; Violet 23, CI 51319.

### Application of the ink composition and preferred processes

The ink composition herein may be applied to the sheet material of the cuff herein by any known method. The ink composition may be applied over the whole surface of the cuff or part thereof. The ink may be applied continuously or intermittently.

It may be applied directly to the nonwoven sheet's surface or via a transfer tool.

It may be applied in a pattern, for example along the edges, or part thereof, of the cuff or of the opening(s) in the cuff, if present.

The ink composition may be applied to the sheet material of the cuff via a transfer tool, which may be any tool, having a surface capable of receiving the ink composition and capable of transferring the ink composition. The transfer tool may have an endless surface, i.e. it may be is in the form of a rotating belt or preferably a roll, such as a printing roll or gravure printing roll (having cavities or cells on its surface, as described below). The transfer tool may comprise a temperature control, e.g. to heat the tool.

The process may involve application of the ink composition onto the surface of the transfer tool by contacting the ink composition and the transfer tool directly or by use of any suitable applicator, for example by use of a spraying system, slot coater, extruder, or optionally even by contacting the surface with a bath comprising the material. Then, the transfer tool with the ink composition on (part of) its surface or in the cells is contacted with the sheet material of the cuff(s) herein. This can be done by moving the articles towards the tool or vise versa or both. Preferred is that the sheet material or the cuffs or the articles herein are supported on another (second) tool, preferably a roll which rotates such that the sheet material or cuffs or articles are rotated and contacted with the transfer tool.

Preferred methods to apply the ink composition, in particular when the ink composition is applied on only parts of the sheet material of the cuff, or even in a specific pattern, comprise printing methods including relief printing, including flexograpghy; planographic printing, which includes offset lithography, screenless lithography, collotype, and waterless printing; intaglio printing, which includes gravure printing, steel-die printing, and copper-plate engraving; stencil printing and screen printing; and electronic printing, which includes electrostatic, magnetographic, ion or electron deposition, and ink-jet printing.

It may be useful to apply the ink composition to the sheet material of the cuff by gravure printing or flexography printing. In flexography printing, the printing element (s) may comprise a raised surface (printing plate) of any pattern or type, e.g. comprising lines, dots, which are then contacted with the ink composition, using for example (a uniformly) engraved or etched roll, e.g. an anilox roll. This roll may pick up the ink composition from a container of any type, and transports/ transfers it unto the printing plate. The ink composition may then be directly transferred from the printing plate to the sheet material of the cuff, thus transferring the ink composition on the raised surfaces (e.g. in a specific pattern) to the nonwoven sheet or cuff. Gravure printing may be done by cutting or engraving or etching opening cells (wells, e.g. in a specific pattern, e.g. lines, dots). The cells may then be flooded and loaded with the ink composition, and if there is any excess ink composition, this may be removed from the surface of the plate (by for example a blade). The ink left in the cells may then be transferred to the sheet material of the cuff. The depth and size of each cell may determine the amount of ink that is transferred to the sheet material. These processes may comprise in addition to the printing pressing step a prepressing step and/ or a post pressing step.

If more than one ink composition is to be applied, this may be done simultaneously or subsequently, typically by the same method but it may be applied by another method.The process herein may comprise as an additional step to apply a further film or coating prior to and/or simultaneously with applying the ink composition, and/ or after applying the ink composition (in the same or a different pattern, but typically such that at least part thereof is present where the ink composition is present).

The process may be such that the ink composition is applied in an amount of at least 0.2 g/m², or at least 0.5 g/m², or at least 01.0g/m², or at least 1.5.g/m² (this being the amount when applied wet or after drying).

The water of the aqueous ink composition is removed after application of the aqueous ink composition, typically by a drying step, as known in the art, for example at temperatures between 50°C and 150°C, preferably 60°C and 100°C or even to 90°C.

This drying step may also act as a curing or annealing step for the possibly film formed by the polymer described herein, resulting in an improved film.

### Test methods used herein:

### Hydrostatic head (hvdrohead)

The hydrostatic head (also referred to as hydrohead) as used herein is measured with a low surface tension liquid, i.e. a 49 mN/m liquid (solution).

This liquid is prepared as set out below.

This test is performed as set out in co-pending application WO2005/112854A, conform the Inda/ Edana test WSP 80.6 (05). However, the water pressure (from below) is increased with a rate is 60 mbar/min.

A sample of 5 cm² is taken from the nonwoven sheet or cuff or topsheet herein (thus, in one embodiment, the nonwoven sheet, cuff or topsheet is such that it has at least an area of 5 cm², or preferably 2.5 x 2.5 cm, that is free of elastic material and edges). The sample should be free from elastic material or edges that are connected to other materials.

The test head used has a 2.5 cm diameter; the protective sleeve used has a 2.2 cm diameter.

The test is performed on this sample and the Hydrostatic head value is obtained, and referred to herein.

### 49 mN/m (dynes/cm) liquid preparation:

A 10 litre canister with tap is cleaned thoroughly 3 times with 2 litres polyethylene and then 3 times with 2 litres distilled/deionized water.

Then, it is filled with 10 litres distilled/deionized water and stirred with a clean stirring bar for 2 h, after which the water is released via the tap.

A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.

The stirring bar is removed from the glass with a magnetic stick (without touching the solution) and then the Na cholate solution is poured into the 10 litres canister and more distilled/deionized water is added such that the concentration of the final solution is 3 g/1. This is further stirred with a stirring bar for 2 hours and then used.

This preparation of the solution and use thereof is at the temperature stated for the test for which it is used, or if no temperature is stated, it is kept at 20°C.

The surface tension of the solution is measured and this should be 49 mN/m (+/- 2). (The surface tension may be determined by method: ASTM D1331-56 ("Standard test method for surface and interfacial tension of solution of surface active agents") using a Kruss K12 tensiometer.)

### Surface tension and surface tension reduction

The surface tension of the ink composition and the reduction thereof when the surface tension reducing agent is added to an ink composition not comprising said agent may be determined by method: ASTM D1331-56 ("Standard test method for surface and interfacial tension of solution of surface active agents") using a Kruss K12 tensiometer.

### Rub-off value

The following method is used herein to determine the rub-off values of the sheet materials or cuffs comprising the ink composition herein.

A sample is cut from the sheet material or cuff comprising the ink composition such that at least the centre thereof comprises the ink composition. The sample may be any size, but as tested herein, a sample comprising at least partially ink composition, of 2.5 by 2.5 cm may be used. If need be, a sample may be attached to a supporting material in order to perform the test (such that the attached supporting material does not impact the test results).

The sample is soaked in distilled water at room temperature for 10 minutes. The sample is then removed from the water and very lightly blotted to remove excess water, with unprinted paper towels.

The sample is mounted on a J.I.S RT-200 Rub Tester (available from DAIEI KAGAKU SEIKI, Kyoto city, Tyoto, Japan)

A receptor material is mounted on the rubbing head of the Rub Tester. The receptor material is shirting #3 test cloth, available from Testfabric Inc., 415 Delaware St., W. Pittston PA USA, Then, following the Rub Tester manufacture's manual the 20-cycles rub-off test, without weight is performed on the samples (i.e. the test parameters are as in the manufacturers manual, based on JIS L0849 method "Color fastness test to rubbing).

The receptor material is then removed from the Rub Tester and let to air-dry at room temperature.

The CIELab colour is then determined by using a LabScan XE, available from HunterLab, with the following settings: Color Scale: CIE L* a* b*; Illuminate: C; Observer: 2°; Port size: 0.5 inch; Illumination Area: 0.5 inch

Delta E between the tested sample and the white, unused receptor material is then measured.

(If more samples can be taken, the test may be repeated and the average Delta E can be calculated, which is as described herein for the Delta E value.)

### Ink optical density

The optical density as referred to herein can be measured via the standard CIELab color and density method, using for example a Gretag Macbeth Specro Eye reflectance spectrophotometer, by placing a sample of the sheet material with ink composition as described herein centered under said spectrophotometer, placed on a white card board (i.e. PG2000, Sun Chemicals, Charlotte, NC, USA). (Settings: 2° observer angle, no filers, ANSI T density standard). (Reference standard method: ASTM E 1331-96, "Standard Test Method for Reflectance Factor and Color by Spectrophotometry Using Hemispherical Geometry".)

### Surface energy

The surface energy of the material referred to herein is measured via the Washburt test, as known in the art, and for example performed by AUGUSTINE SCIENTIFIC, LLC , Newbury, Ohio, USA. (If a low surface tension liquid of 49 mN/m is used, it may be prepared as set out above in the Hydrostatic head test.)

While particular embodiments of the present invention have been illustrated and described, it would be obvious to those skilled in the art that various other changes and modifications that are within the scope of this invention. Each embodiment defined by certain properties or dimension for which a value is defined herein is to be understood to include embodiments with functional equivalent properties or dimensions, e.g. a dimension of 0.5 cm has to be understood as meaning "about 0.5 cm".

## Claims

1. Process for making an absorbent article comprising a cuff, wherein the cuff is a leg cuff, a barrier cuff, an anal cuff or a genital cuff, the cuff comprising a nonwoven sheet material that comprises an ink composition, including the steps of:
a) obtaining an hydrophobic nonwoven sheet material having a hydrostatic head of at least 15 mbar (as measured according to the hydrostatic head test described herein);
b) obtaining an aqueous ink composition comprising water, a pigment, a fixation aid polymer which is an elastic film-forming polymer, and a surface tension reducing agent;
c) forming said nonwoven sheet material into a cuff and applying the cuff to an absorbent article, or applying said nonwoven sheet material to an absorbent article and forming it into a cuff, to obtain an absorbent article with a cuff;
d) applying said ink composition to said nonwoven sheet of step a) or to said cuff of step c), and
e) drying said nonwoven or cuff of step d).

2. A process as in the preceding claim, whereby said fixation aid polymer is in the form of a dispersion of the polymer in water.

3. A process according to claim 1 or 2, wherein the ink composition comprises less than 5% by weight of an organic solvent.

4. A process according to any of the preceding claims, whereby said ink composition comprises a surface tension reducing agent, capable of reducing the surface tension of the aqueous ink composition with at least 8 mN/m (as measured with ASTM D1331-56).

5. A process according to any of the preceding claims, whereby said nonwoven sheet material has a hydrostatic head value of at least 20 mbar.

6. A process according to any of the preceding claims, whereby said ink composition is applied in the form of an aqueous dispersion or solution comprising at least 30% by weight of water and less than 10% by weight, or preferably less than 5%, by weight, of an organic solvent.

7. A process according to any of the preceding claims, whereby said cuff is an anal and/or a genital cuff, whereby said cuff comprises an opening with longitudinal edges that has along each said edge at least one elastic band extending in longitudinal direction along said opening and beyond said opening, said ink composition being present along and/ or in close proximity to one or more of said edges of the opening and/ or along or in close proximity to said elastic band or bands.

8. A process according to any of the preceding claims, whereby said ink composition comprising a surface tension reducing agent, which comprises a diol-surfactant, preferably selected from dioctyl sodium sulfosuccinate or derivatives thereof, ethoxylated glycols, sorbitan esters and/ or acetylenic diol-based surfactants and mixtures thereof.

## Patentansprüche

1. Verfahren zur Herstellung eines Absorptionsartikels, der ein Bündchen umfasst, wobei das Bündchen ein Beinbündchen, ein Sperrbündchen, ein Analbereichsbündchen oder ein Genitalbereichsbündchen ist, wobei das Bündchen ein Flächengebilde aus Vliesmaterial umfasst, das eine Tintenzusammensetzung umfasst, die folgenden Schritte aufweisend:
a) Beschaffen eines hydrophoben Flächengebildes aus Vliesmaterial mit einem hydrostatischen Druck von mindestens 15 mbar (gemessen gemäß dem hierin beschriebenen hydrostatischen Drucktest);
b) Erhalten einer wässrigen Tintenzusammensetzung, die Wasser, ein Pigment, ein Fixierhilfepolymer, bei dem es sich um ein Polymer handelt, das einen elastischen Film bilden kann, und ein Mittel, das eine Oberflächenspannung herabsetzt, umfasst;
c) Ausbilden eines Bündchens aus dem Flächengebilde aus Vliesmaterial und Aufbringen des Bündchens auf einen Absorptionsartikel, oder Aufbringen des Flächengebildes aus Vliesmaterial auf einen Absorptionsartikel und Ausbilden eines Bündchens daraus, um einen Absorptionsartikel mit einem Bündchen zu erhalten;
d) Aufbringen der Tintenzusammensetzung auf das Flächengebilde aus Vliesmaterial von Schritt a) oder auf das Bündchen von Schritt c) und
e) Trocknen des Vliesmaterials oder des Bündchens von Schritt d).

2. Verfahren nach dem vorgenannten Anspruch, wobei das Fixierhilfepolymer in Form einer Dispersion des Polymers in Wasser vorliegt.

3. Verfahren nach Anspruch 1 oder 2, wobei die Tintenzusammensetzung weniger als 5 Gew.-% an organischem Lösungsmittel umfasst.

4. Verfahren nach einem der vorgenannten Ansprüche, wobei die Tintenzusammensetzung ein Mittel zur Herabsetzung einer Oberflächenspannung umfasst, das in der Lage ist, die Oberflächenspannung der wässrigen Tintenzusammensetzung mit mindestens etwa 8 mN/m (gemessen mit ASTM D1331-56) herabzusetzen.

5. Verfahren nach einem der vorgenannten Ansprüche, wobei das Flächengebilde aus Vliesmaterial einen hydrostatischen Druckwert von mindestens 20 mbar aufweist.

6. Verfahren nach einem der vorstehenden Ansprüche, wobei die Tintenzusammensetzung in Form einer wässrigen Dispersion oder Lösung aufgebracht wird, die mindestens 30 Gew.-% Wasser und weniger als 10 Gew.-% oder vorzugsweise weniger als 5 Gew.-% organisches Lösungsmittel enthält.

7. Verfahren nach einem der vorstehenden Ansprüche, wobei das Bündchen ein Analbereichs- oder Genitalbereichsbündchen ist, wobei das Bündchen eine Öffnung mit Längsrändern umfasst, die entlang jedes dieser Ränder mindestens ein elastisches Band aufweist, das sich in Längsrichtung entlang der Öffnung und über die Öffnung hinaus erstreckt, wobei die Tintenzusammensetzung entlang oder in großer Nähe eines oder mehrerer von den Rändern der Öffnung und/oder entlang oder in großer Nähe des mindestens einen elastischen Bandes vorhanden ist.

8. Verfahren nach einem der vorstehenden Ansprüche, wobei die Tintenzusammensetzung ein Mittel zur Herabsetzung einer Oberflächenspannung umfasst, das ein Dioltensid umfasst, das vorzugsweise ausgewählt ist aus Dioctylnatriumsulfosuccinat oder Derivaten davon, ethoxylierten Glycolen, Sorbitanestern und/oder Tensiden auf Basis von Acetylendiol und Mischungen davon.

## Revendications

1. Procédé de fabrication d'un article absorbant comprenant un revers, dans lequel le revers est un revers de jambe, un revers formant barrière, un revers anal ou un revers génital, le revers comprenant un matériau de feuille non tissée qui comprend une composition d'encre, incluant les étapes consistant à :
a) obtenir un matériau de feuille non tissée hydrophobe ayant une charge hydrostatique d'au moins 15 mbar (telle que mesurée selon le test de charge hydrostatique décrit ici) ;
b) obtenir une composition aqueuse d'encre comprenant de l'eau, un pigment, un polymère auxiliaire de fixation qui est un polymère filmogène élastique, et un agent réducteur de tension superficielle ;
c) former ledit matériau de feuille non tissée en un revers et appliquer le revers sur un article absorbant, ou appliquer ledit matériau de feuille non tissée sur un article absorbant et le former en un revers, pour obtenir un article absorbant avec un revers ;
d) appliquer ladite composition d'encre sur ladite feuille non tissée de l'étape a) ou sur ledit revers de l'étape c), et
e) sécher ledit non-tissé ou revers de l'étape d).

2. Procédé selon la revendication précédente, dans lequel ledit polymère auxiliaire de fixation se présente sous la forme d'une dispersion du polymère dans l'eau.

3. Procédé selon la revendication 1 ou 2, dans lequel la composition d'encre comprend moins de 5 % en poids d'un solvant organique.

4. Procédé selon l'une quelconque des revendications précédentes, selon lequel ladite composition d'encre comprend un agent réducteur de tension superficielle, susceptible de réduire la tension superficielle de la composition aqueuse d'encre d'au moins environ 8 mN/m (tel que mesuré avec l'ASTM D1331-56).

5. Procédé selon l'une quelconque des revendications précédentes, selon lequel ledit matériau de feuille non tissée a une valeur de charge hydrostatique d'au moins 20 mbar.

6. Procédé selon l'une quelconque des revendications précédentes, selon lequel ladite composition d'encre est appliquée sous la forme d'une dispersion ou solution aqueuse comprenant au moins 30 % en poids d'eau et moins de 10 % en poids, ou de préférence moins de 5 % en poids, d'un solvant organique.

7. Procédé selon l'une quelconque des revendications précédentes, selon lequel ledit revers est un revers anal et/ou génital, selon lequel ledit revers comprend une ouverture avec des bords longitudinaux qui a le long de chaque bord précité au moins une bande élastique s'étendant dans une direction longitudinale le long de ladite ouverture et au-delà de ladite ouverture, ladite composition d'encre étant présente le long et/ou à proximité immédiate d'un ou plusieurs desdits bords d'ouverture et/ou le long ou à proximité immédiate de ladite bande élastique ou desdites bandes élastiques.

8. Procédé selon l'une quelconque des revendications précédentes, selon lequel ladite composition d'encre comprend un agent réducteur de tension superficielle, qui comprend un agent tensioactif diol, de préférence choisi parmi le sulfosuccinate sodique de dioctyle ou des dérivés de celui-ci, des glycols éthoxylés, des esters de sorbitan et/ou des agents tensioactifs acétylénique à base de diol et leurs mélanges.
